(19) Europäisches Patentamt
European Patent Office
Office européen des brevets

(11) **EP 3 888 549 A1**

(12) **EUROPÄISCHE PATENTANMELDUNG**

(43) Veröffentlichungstag:
06.10.2021 Patentblatt 2021/40

(51) Int Cl.:
*A61B 5/113* (2006.01)

(21) Anmeldenummer: 20167693.9

(22) Anmeldetag: 02.04.2020

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: BIOTRONIK SE & Co. KG
12359 Berlin (DE)

(72) Erfinder: SKERL, Olaf
18209 Bad Doberan (DE)

(74) Vertreter: Biotronik Corporate Services SE
Corporate Intellectual Property
Sieversufer 7-9
12359 Berlin (DE)

(54) **BESTIMMUNG DER ATMUNG MIT HILFE EINES BESCHLEUNIGUNGSSENSORS**

(57) Die Erfindung betrifft ein medizinisches Gerät (1), mit einem Beschleunigungssensor (2) zum Erfassen einer Atmung einer Person (P), der dazu ausgebildet ist, einen momentanen dreidimensionalen Beschleunigungsvektor ($a(t)$) zu messen, und einer Auswertungseinheit (3), die dazu ausgebildet ist, aus einer Änderung der Winkellage des momentanen Beschleunigungsvektors bezüglich eines Referenzvektors ($a_0$) zumindest einen die Atmung der Person (P) charakterisierenden Parameter zu bestimmen, wenn der Beschleunigungssensor (2) an oder in einem Oberkörper der Person (P) angeordnet ist.

FIG. 1

**Beschreibung**

Die vorliegende Erfindung betrifft ein medizinisches Gerät, insbesondere ein implantierbares medizinisches Gerät, zum Bestimmen eines die Atmung eines Patienten betreffenden Parameters (z. B. eine Atemfrequenz) sowie ein entsprechendes Verfahren zum Bestimmen eines solchen Parameters.

Viele physiologische Parameter sind von der Atmung abhängig oder werden von ihr beeinflusst. Darüber hinaus ist die Atmung einer Person bzw. eines Patienten oft selbst ein diagnostischer Parameter (z. B. Schlafapnoe). Die Bestimmung der Atmung ist oftmals notwendig, um den Atmungseinfluss auf weitere diagnostische Parameter abschätzen zu können.

Bekannte Lösungen zur Atmungsbestimmung beinhalten z. B. eine direkte Bestimmung der Atmung über eine Messung der Luftströmung. Weiterhin sind indirekte Atmungsbestimmungen über den Atmungseinfluss auf physiologische Größen bekannt (z. B. Herzrate, Blutdrucke etc.).

Die US 2009/0062628 A1 beschreibt eine Schlafdiagnosevorrichtung, die einen dreidimensionalen Beschleunigungssensor und einen Haltungsdetektor aufweist, der dazu konfiguriert ist, eine Haltung eines Patienten aus einer Gleichstromkomponente des dreidimensionalen Beschleunigungssensor zu detektieren. Weiterhin weist die Schlafdiagnosevorrichtung einen Atembewegungsmelder auf, der dazu konfiguriert ist, eine Atembewegung des Patienten aus einer Wechselstromkomponente des dreidimensionalen Beschleunigungssensors zu detektieren.

Weiterhin offenbart die US 2015/0342518 A1 ein Verfahren zum Berechnen eines gefilterten Atemsignals für ein Atemübungsgerät.

Nachteilig an den bekannten Lösungen ist insbesondere, dass eine direkte Messung der Luftströmung verhältnismäßig aufwendig ist und oftmals nur temporär möglich ist. Zudem ist die Ableitung der Atmung aus physiologischen Parametern häufig fehlerbehaftet aufgrund dritter Einflüsse auf derartige Parameter.

Der vorliegenden Erfindung liegt daher die Aufgabe zugrunde, eine Bestimmung der Atmung mittels eines medizinischen Geräts bereitzustellen, die auf einer nichtphysiologischen Messgröße beruht und ohne eine direkte Messung des Luftstromes auskommt.

Diese Aufgabe wird durch ein medizinisches Gerät mit den Merkmalen des Anspruchs 1 sowie durch ein Verfahren mit den Merkmalen des Anspruchs 8 gelöst. Vorteilhafte Ausgestaltungen des jeweiligen Erfindungsaspekts sind in den entsprechenden Unteransprüchen angegeben.

Gemäß Anspruch 1 wird ein medizinisches Gerät offenbart, mit: einem Beschleunigungssensor zum Erfassen einer Atmung einer Person bzw. eines Patienten, wobei der Beschleunigungssensor dazu ausgebildet ist, einen momentanen dreidimensionalen Beschleunigungsvektor zu messen, und mit einer Auswertungseinheit, die dazu ausgebildet ist, aus einer Änderung der Winkellage des momentanen Beschleunigungsvektors bezüglich eines raumfesten Referenzvektors zumindest einen die Atmung der Person charakterisierenden Parameter zu bestimmen, insbesondere wenn der Beschleunigungssensor an oder in einem Oberkörper der Person angeordnet ist.

Die Atmung wird also mit anderen Worten insbesondere aus dem Signal eines 3D-Beschleunigungssensors gewonnen, der sich im oder am Oberkörper des Patienten befindet, und zwar vorzugsweise nur durch Analyse der Ausgangssignale dieses Sensors.

Gemäß einer bevorzugten Ausführungsform des medizinischen Geräts ist die Auswertungseinheit dazu ausgebildet, zum Erfassen der Änderung der Winkellage eine der folgenden Größen wiederholt zu bestimmen: einen momentanen Winkel $\delta(t)$ zwischen dem momentanen Beschleunigungsvektor $a(t)$ und dem Referenzvektor $a_0$; den Kosinus [$\cos\delta(t)$] eines momentanen Winkels $\delta(t)$ zwischen dem momentanen Beschleunigungsvektor $a(t)$ und dem Referenzvektor $a_0$; das Quadrat des Kosinus [$(\cos\delta(t))^2$] eines momentanen Winkels $\delta(t)$ zwischen dem momentanen Beschleunigungsvektor $a(t)$ und dem Referenzvektor $a_0$.

Die Änderung der Winkellage bzw. die wiederholt bestimmte Größe entspricht also einer insbesondere periodischen Atmungskurve der Person über der Zeit, anhand derer z. B. eine Atemfrequenz der Person bestimmbar ist.

Weiterhin ist gemäß einer Ausführungsform des medizinischen Geräts vorgesehen, dass die Auswertungseinheit dazu ausgebildet ist, zum Bestimmen der jeweiligen Größe ein Skalarprodukt zwischen dem momentanen Beschleunigungsvektor $a(t)$ und dem Referenzvektor $a_0$ zu berechnen.

Dieses Skalarprodukt $a(t) \cdot a_0$ kann normiert werden und ergibt dann den besagten Kosinus des momentanen Winkels $\delta(t)$ gemäß:

$$\cos\delta(t) = \frac{a(t) \cdot a_0}{|a(t)||a_0|} = \frac{a_x(t)a_{x0} + a_y(t)a_{y0} + a_z(t)a_{z0}}{\sqrt{a_x^2(t) + a_y^2(t) + a_z^2(t)}\sqrt{a_{x0}^2 + a_{y0}^2 + a_{z0}^2(t)}}.$$

Der momentane Winkel kann durch die entsprechende Umkehrfunktion bestimmt werden:

$$\delta(\mathrm{t}) = \cos^{-1}\left[\frac{a(t)\cdot a_0}{|a(t)||a_0|}\right] = \cos^{-1}\left[\frac{a_x(t)a_{x0}+a_y(t)a_{y0}+a_z(t)a_{z0}}{\sqrt{a_x^2(t)+a_y^2(t)+a_z^2(t)}\sqrt{a_{x0}^2+a_{y0}^2+a_{z0}^2(t)}}\right].$$

Um den Rechenaufwand (und damit Energiebedarf) zu reduzieren, muss für die Atmungsbestimmung der Winkel $\delta(t)$ nicht zwingend bestimmt werden, die Verwendung des $\cos \delta(t)$ ist hierfür ausreichend.

Besonders bei Anwendungen, die keine großen Rechen- bzw. Energiekapazitäten aufweisen (z. B. bei einem medizinischen Gerät in Form eines implantierbaren medizinischen Geräts), erweist sich die Verwendung des Quadrats des Kosinus als vorteilhaft, da eine verhältnismäßig aufwendige Abschätzung der Wurzelterme entfallen kann:

$$(\cos\delta(\mathrm{t}))^2 = \frac{\left(a_x(t)a_{x0}+a_y(t)a_{y0}+a_z(t)a_{z0}\right)^2}{\left(a_x^2(t)+a_y^2(t)+a_z^2(t)\right)\left(a_{x0}^2+a_{y0}^2+a_{z0}^2(t)\right)}.$$

Es hat sich gezeigt, dass auch diese Größe eine periodische Atmungskurve darstellt, aus der der besagte Parameter (z. B. Atemfrequenz) ermittelbar ist.

Gemäß einer Ausführungsform des medizinischen Geräts ist der Beschleunigungssensor dazu ausgebildet, den Beschleunigungsvektor mit einer Abtastrate im Bereich von 10 Hz bis 100 Hz abzutasten.

Weiterhin ist gemäß einer Ausführungsform des medizinischen Geräts vorgesehen, dass der Beschleunigungssensor hinsichtlich des Betrags des Beschleunigungsvektors eine Auflösung im Bereich von 0,001 m/s$^2$ bis 0,01 m/s$^2$ aufweist.

Weiterhin ist gemäß einer Ausführungsform des medizinischen Geräts vorgesehen, dass die Auswertungseinheit dazu ausgebildet ist, die Ausgangssignale des Beschleunigungssensors $a_x(t)$, $a_y(t)$, $a_z(t)$, die den momentanen Beschleunigungsvektor $a(t)$ angeben vor und/oder nach der Bestimmung der besagten Größe (z.B. $\cos \delta(t)$ oder $(\cos \delta(t))^2$) zu filtern.

Als Referenzvektor $a_0$ kann grundsätzlich gemäß einer Ausführungsform des medizinischen Geräts ein beliebiger fester Referenzvektor verwendet werden.

Weiterhin ist gemäß einer Ausführungsform des medizinischen Geräts vorgesehen, dass der Referenzvektor einer der folgenden Vektoren ist: ein bei einer Initialisierung des medizinischen Geräts bestimmter Vektor (z. B. aus den Daten des Beschleunigungssensors, vorzugsweise ist ein bei der Initialisierung bestimmter Vektor senkrecht zum dabei gemessenen Beschleunigungsvektor), ein zu bestimmten Zeitpunkten aus Daten des Beschleunigungssensors bestimmter Vektor, oder ein willkürlich festgelegter Vektor.

Weiterhin ist gemäß einer Ausführungsform des medizinischen Geräts vorgesehen, dass es sich bei dem mindestens einen Parameter um einen der folgenden Parameter handelt: eine Atemfrequenz, eine zeitliche Länge einer Atemperiode, insbesondere die Zeit zwischen zwei Einatmungen, eine Schwankung einer zeitlichen Länge der Atemperiode, eine zeitliche Länge einer Atempause.

Insbesondere kann die Auswertungseinheit dazu ausgebildet sein, die besagte Größe (z. B. Winkel, Kosinus des Winkels, oder Quadrat des Kosinus des Winkels) bzw. die Atmungskurve auf Unregelmäßigkeiten der Atmung (z. B. Schlafapnoe) hin zu analysieren, beispielsweise durch die Bestimmung der Abstände zwischen zwei Einatmungszyklen.

Gemäß einer Ausführungsform der Erfindung ist das medizinische Gerät dazu ausgebildet, an die Person eine Therapie abzugeben, z. B. in Form einer elektrischen Stimulation, insbesondere in Form einer elektrischen Stimulation des Herzens oder in Form einer Neurostimulation, wobei die Therapie in Abhängigkeit des mindestens einen Parameters durch das medizinische Gerät automatisch verändert und/oder eingeleitet wird.

Weiterhin ist das medizinische Gerät gemäß einer Ausführungsform der Erfindung dazu ausgebildet, eine weiterhin erfasste physiologische Messgröße des Patienten (z. B. eine Herzrate oder einen Pulmonalarteriendruck (PA-Druck)) mittels des mindestens einen Parameters (siehe oben) zu korrigieren und/oder mit dem mindestens einen Parameter zu korrelieren (oder anderweitig zu kombinieren).

Gemäß einer bevorzugten Ausführungsform des medizinischen Geräts ist vorgesehen, dass das medizinische Gerät ein implantierbares medizinisches Gerät ist, das vorzugsweise dazu ausgebildet ist, in einen Oberkörper der Person implantiert zu werden.

Weiterhin ist gemäß einer bevorzugten Ausführungsform des medizinischen Geräts vorgesehen, dass das medizinische Gerät ein implantierbares Gehäuse aufweist, wobei der Beschleunigungssensor und/oder die Auswertungseinheit in einem durch das Gehäuse gebildeten Innenraum angeordnet ist bzw. sind. Weiterhin dichtet das Gehäuse vorzugsweise den Beschleunigungssensor und/oder die Auswertungseinheit hermetisch ab. Das medizinische Gerät kann z.B. eines der folgenden medizinischen Geräte sein: ein diagnostisches medizinisches Gerät (z. B. ein Patch (sog.

diagnostisches Pflaster), ein implantierbarer Drucksensor, ein implantierbares diagnostisches Gerät, insbesondere zur Aufzeichnung von Fernfeldelektrokardiogrammen (auch bekannt als *loop recorder*), ein implantierbarer Herzschrittmacher, ein implantierbarer Kardioverter-Defibrillator, oder ein implantierbarer Neuromstimulator).

Gemäß einem weiteren Aspekt der vorliegenden Erfindung wird ein Verfahren zum Erfassen einer Atmung einer Person unter Verwendung eines erfindungsgemäßen medizinischen Gerätes offenbart, wobei das Verfahren die Schritte aufweist:

- Wiederholtes Messen eines momentanen Beschleunigungsvektors ($a$(t)) mittels des Beschleunigungssensors, und
- Bestimmen zumindest eines die Atmung der Person charakterisierenden Parameters aus einer Änderung der Winkellage des momentanen Beschleunigungsvektors bezüglich eines raumfesten Referenzvektors, wenn der Beschleunigungssensor an oder in einem Oberkörper eines Patienten angeordnet ist.

Bei dem Parameter kann es sich um zumindest einen der oben genannten Parameter handeln (z. B. Atemfrequenz etc.).

Gemäß einer Ausführungsform des Verfahrens wird zum Erfassen der Änderung der Winkellage des dreidimensionalen Beschleunigungsvektors eine der folgenden Größen mittels der Auswertungseinheit wiederholt bestimmt: ein momentaner Winkel $\delta$(t) zwischen dem momentanen Beschleunigungsvektor $a$(t) und dem Referenzvektor $a_0$; ein Kosinus [$\cos\delta$(t)] des momentanen Winkels $\delta$(t) zwischen dem momentanen Beschleunigungsvektor $a$(t) und dem Referenzvektor $a_0$; ein Quadrat des Kosinus [$(\cos\delta(t))^2$] [$\cos^2\delta(t)$] des momentanen Winkels $\delta$(t) zwischen dem momentanen Beschleunigungsvektor $a$(t) und dem Referenzvektor $a_0$.

Weiterhin ist gemäß einer Ausführungsform des Verfahrens vorgesehen, dass mittels der Auswertungseinheit zum Bestimmen der jeweiligen Größe ein Skalarprodukt (siehe oben) zwischen dem momentanen Beschleunigungsvektor $a$(t) und dem Referenzvektor $a_0$ wiederholt berechnet wird.

Ein weiterer Aspekt der Erfindung betrifft ein Computerprogramm aufweisend Befehle, die bewirken, dass das medizinische Gerät nach einem der Ansprüche 1 bis 7 die Verfahrensschritte des Verfahrens nach einem der Ansprüche 8 bis 10 ausführt.

Ein weiterer Aspekt der Erfindung betrifft ein Medium, auf dem das erfindungsgemäße Computerprogramm gespeichert ist.

Im Folgenden sollen Ausführungsformen der Erfindung sowie weitere Merkmale und Vorteile der Erfindung anhand der Figuren erläutert werden. Es zeigen:

Fig. 1 eine Ausführungsform eines erfindungsgemäßen medizinischen Geräts in Form eines implantierbaren Loop-Recorders;

Fig. 2 eine Darstellung des Kosinus des zeitlichen Verlaufs des Winkels zwischen dem momentanen Beschleunigungsvektor und einem Referenzvektor (z. B. der Vektor der Gravitation); und

Fig. 3 eine Darstellung des Quadrats des Kosinus des zeitlichen Verlaufs des Winkels zwischen dem momentanen Beschleunigungsvektor und einem Referenzvektor (z. B. der Vektor der Gravitation).

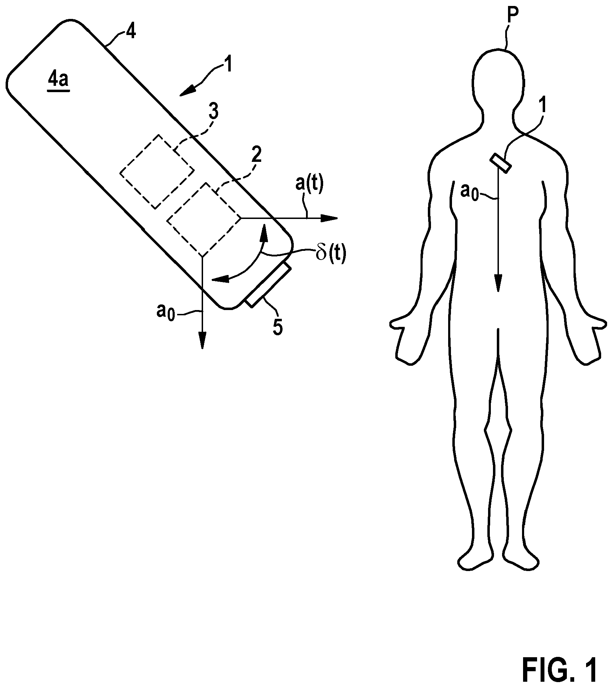

Figur 1 zeigt eine schematische Darstellung einer Ausführungsform eines erfindungsgemäßen medizinischen Geräts 1, wobei es sich bei dem Gerät 1 z. B. um einen implantierbaren Loop-Recorders 1 handelt, der ein hermetisches Gehäuse 4 aufweist, das einen Innenraum 4a umgibt. Das Gehäuse 4 bzw. das Implantat 1 ist z. B. subkutan in der Nähe des Brustbeins oder auf einem Brustmuskel einer Person P bzw. eines Patienten P implantierbar und kann zur Aufnahme von elektrischen Signalen zumindest eine Elektrode 5 an einem Ende des Gehäuses 4 aufweisen. Bei dem Gerät 1 kann es sich jedoch auch um ein anderes Implantat handeln (siehe unten), beispielsweise ein Herzschrittmacher (subkutan oder intrakardial), bei welchem die Elektrode 5 zu Abgabe von elektrischen Stimulationsimpulsen eingerichtet und vorgesehen ist.

Zum Erfassen einer Atmung der Person P weist das implantierbare medizinzische Gerät 1 einen Beschleunigungssensor 2 auf, der dazu ausgebildet ist, einen momentanen dreidimensionalen Beschleunigungsvektor $a$(t) des Geräts 1 bzw. des Gehäuses 4 zu messen, sowie eine Auswertungseinheit 3, die dazu ausgebildet ist, aus einer Änderung der Winkellage des momentanen Beschleunigungsvektors bezüglich eines Referenzvektors $a_0$ zumindest einen die Atmung der Person P charakterisierenden Parameter zu bestimmen. Gemäß Figur 1 sind der Beschleunigungssensor 2 und die Auswertungseinheit 3 bevorzugt im Innenraum 4a des Gehäuses 4 angeordnet und durch das Gehäuse 2 hermetisch abgedichtet.

Atmungsbedingt senkt und hebt sich der Brustkasten der Person P, was zu der besagten periodischen Änderung der Winkellage des Beschleunigungssensors 2 und damit des gemessenen Beschleunigungsvektors $a$(t) bezüglich des Referenzvektors $a_0$ führt, der hier z. B. durch den Gravitationsvektor gegeben ist. D. h., der Winkel zwischen dem momentanen Beschleunigungsvektor **a**(t) und dem Referenzvektor **a**$_0$ ändert sich atmungsbedingt.

Das erfindungsgemäße Prinzip kann auf alle erdenklichen medizinischen Geräte angewendet werden, die einen dreidimensionalen Beschleunigungssensor aufweisen, der an oder im Oberkörper einer Person P bzw. eines Patienten P positionierbar ist. Anstelle eines Herzschrittmachers kann es sich bei dem Gerät 1 auch um ein diagnostisches Gerät (z. B. um einen Patch, einen Loop Recorder, oder einen implantierbaren Drucksensor) oder um ein anderweitiges Therapiegerät handeln (z. B. einen Defibrillator-Kardioverter oder einen Neurostimulator), das auf dem Oberkörper der Person P anbringbar oder in den Oberkörper der Person P implantierbar ist. Diese Geräte verfügen mit Vorteil in der Regel bereits über einen 3D-Beschleunigungssensor, der den aktuellen Vektor der Beschleunigung **a**(t) liefert.

Die durch die Atmung verursachten Beschleunigungen sind in der Regel jedoch zu gering und können mit einem Beschleunigungssensor nicht direkt gemessen werden. Zusätzlich sind die Signale durch andere Bewegungen (z. B. Herzschlag) stark überlagert.

Der Erfindung liegt daher das überraschend gut funktionierende Prinzip zugrunde, wonach die Bestimmung der Atmung auf den geringfügigen Änderungen der Winkellage des 3D-Beschleunigungssensors 2 durch die Atembewegungen beruht.

Diesbezüglich ist es bekannt, dass der Winkel δ(t) zwischen zwei Vektoren (hier der momentane Beschleunigungsvektor **a**(t) und der Referenzvektor **a**$_0$) mittels des Skalarproduktes der Vektoren bestimmt werden kann:

$$\cos\delta(t) = \frac{\mathbf{a}(t)\cdot\mathbf{a}_0}{|\mathbf{a}(t)||\mathbf{a}_0|} = \frac{a_x(t)a_{x0}+a_y(t)a_{y0}+a_z(t)a_{z0}}{\sqrt{a_x^2(t)+a_y^2(t)+a_z^2(t)}\sqrt{a_{x0}^2+a_{y0}^2+a_{z0}^2(t)}}.$$

Damit kann die aktuelle, zeitabhängige Winkellage des Beschleunigungssensors 2 als Winkel δ(t) zum Referenzvektor **a**$_0$ bestimmt werden.

Als Referenzvektor **a**$_0$ kann hierbei ein beliebiger fester Vektor verwendet werden, also z. B. ein bei einer Initialisierung bestimmter Vektor (z. B. aus den Daten des 3D-Beschleunigungssensors 2) oder ein zu bestimmten Zeitpunkten aus den Daten des 3D-Beschleunigungssensors 2 bestimmter Vektor. In der Figur 1 ist als Beispiel der Vektor der Gravitation bei aufrechter Körperlage der Person als Referenzvektor **a**$_0$ angegeben.

Um den Rechenaufwand (und damit Energiebedarf) zu reduzieren, muss für die Atmungsbestimmung der Winkel δ(t) nicht zwingend bestimmt werden, die Verwendung des cos δ(t) ist hierfür ausreichend.

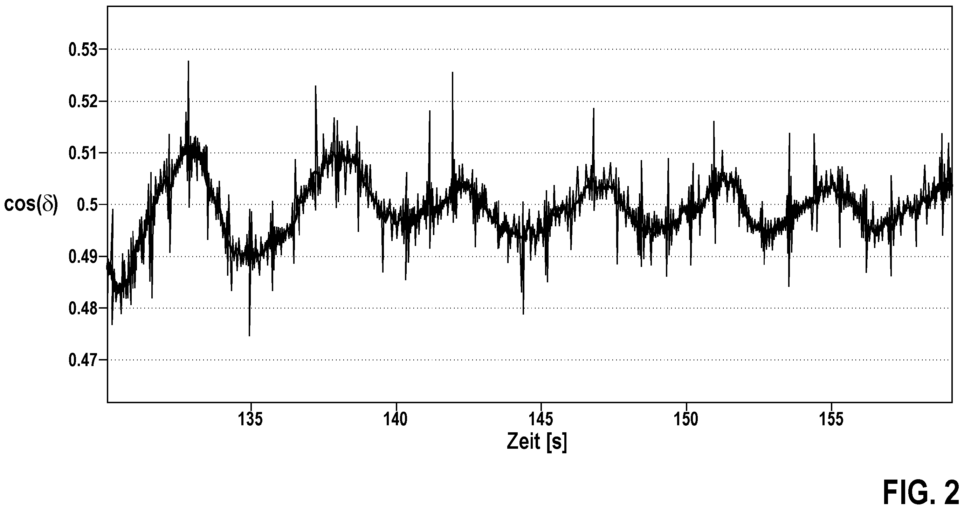

Bei ausreichend hoher Abtastrate (z.B. 10 Hz bis 100 Hz) und Auflösung (0,001 m/s$^2$ bis 0,01 m/s$^2$) des 3D-Beschleunigungssignals erhält man den Verlauf der durch die Atmung verursachten Winkelabweichung des Beschleunigungssensors 2 (Atmungskurve cos δ(t)). Dieses Signal ist in der Fig. 2 gezeigt. Die Signale des Beschleunigungssensors 2 können vor und/oder nach der Winkelbestimmung geeignet gefiltert werden.

Aus der Atmungskurve gemäß Fig. 2 können automatisch mittels der Auswertungseinheit 3 geeignete Parameter bestimmt werden, die für die Charakterisierung der Atmung verwendet werden können (z. B. Atemfrequenz, Länge einer Atemperiode, Schwankung der Länge einer Atemperiode, Atempausen etc.). Die Auswertungseinheit 3 kann hierzu z. B. zumindest einen geeigneten integrierten Schaltkreis aufweisen.

Mittels der Analyse der Atmungskurve können mögliche Unregelmäßigkeiten der Atmung (z. B. Schlafapnoe) bestimmt werden. Anhand geeigneter Parameter der Atmungskurve können Therapieoptionen automatisch optimiert oder Therapien eingeleitet werden.

Erfasste diagnostische Parameter (Herzrate, PA-Druck etc.) können mit der Atmung korrigiert oder korreliert oder kombiniert werden.

Für eine weitere Reduzierung des Rechenaufwandes und damit des Energiebedarfs kann auch das Quadrat des cos δ(t) verwendet werden. Der Wegfall des Radizierens ist besonders für implantierbare medizinische Geräte sinnvoll:

$$(\cos\delta(t))^2 = \frac{\left(a_x(t)a_{x0}+a_y(t)a_{y0}+a_z(t)a_{z0}\right)^2}{\left(a_x^2(t)+a_y^2(t)+a_z^2(t)\right)\left(a_{x0}^2+a_{y0}^2+a_{z0}^2(t)\right)}.$$

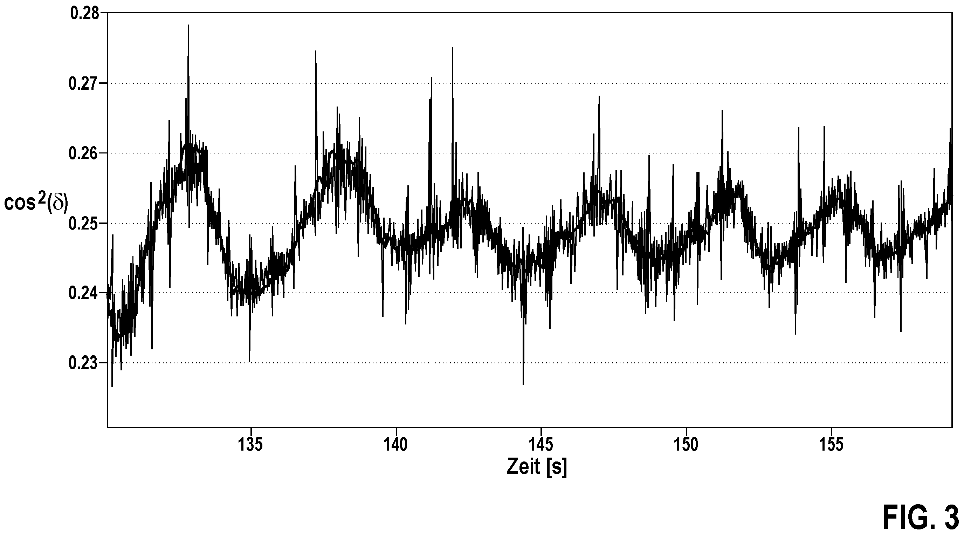

Die o.g. Parameter lassen sich auch aus den mit dieser weiter vereinfachten Methode berechneten Atmungskurve (vgl. Fig. 3) bestimmen.

Die vorliegende Erfindung erweist sich als vorteilhaft, da sie mit geringem Aufwand und insbesondere unter Ausnutzung eines (möglicherweise ohnehin bereits vorhandenen) kostengünstigen Beschleunigungssensors die Atmungsbestimmung erlaubt, und zwar im Wesentlichen ohne Beeinträchtigung der Person bzw. des Patienten. Aufgrund der Erfindung ist hinsichtlich der Atmung eine kontinuierliche Messung möglich, so dass sich die Erfindung insbesondere für eine Langzeitüberwachung eignet. Darüber hinaus weisen die oben vorgestellten Algorithmen einen vergleichsweise geringen Energiebedarf auf. Schließlich ermöglicht die Erfindung die Erweiterung des Funktionsumfangs vieler medizinischer Geräte und gestattet mit Vorteil eine Einbeziehung der Atmung in die Diagnostik / Therapie / Therapieanpassung (z. B. Reaktion auf Schlafapnoe).

**Patentansprüche**

1. Medizinisches Gerät (1), mit:

    - einem Beschleunigungssensor (2) zum Erfassen einer Atmung einer Person (P), der dazu ausgebildet ist, einen momentanen dreidimensionalen Beschleunigungsvektor ($a$(t)) zu messen, und
    - einer Auswertungseinheit (3), die dazu ausgebildet ist, aus einer Änderung der Winkellage des momentanen Beschleunigungsvektors ($a$(t)) bezüglich eines Referenzvektors ($a_0$) zumindest einen die Atmung der Person (P) charakterisierenden Parameter zu bestimmen, wenn der Beschleunigungssensor (2) an oder in einem Oberkörper der Person (P) angeordnet ist.

2. Medizinisches Gerät nach Anspruch 1, **dadurch gekennzeichnet, dass** die Auswertungseinheit (3) dazu ausgebildet ist, zum Erfassen der Änderung der Winkellage, eine der folgenden Größen wiederholt zu bestimmen: einen momentanen Winkel $\delta$(t) zwischen dem momentanen Beschleunigungsvektor ($a$(t)) und dem Referenzvektor ($a_0$), den Kosinus eines momentanen Winkels $\delta$(t) zwischen dem momentanen Beschleunigungsvektor ($a$(t)) und dem Referenzvektor ($a_0$), das Quadrat des Kosinus eines momentanen Winkels $\delta$(t) zwischen dem momentanen Beschleunigungsvektor ($a$(t)) und dem Referenzvektor ($a_0$).

3. Medizinisches Gerät nach Anspruch 1 oder 2, **dadurch gekennzeichnet, dass** die Auswertungseinheit (3) dazu ausgebildet ist, zum Bestimmen der jeweiligen Größe ein Skalarprodukt zwischen dem momentanen Beschleunigungsvektor ($a$(t)) und dem Referenzvektor ($a_0$) zu berechnen.

4. Medizinisches Gerät nach einem der vorhergehenden Ansprüche, **dadurch gekennzeichnet, dass** der Referenzvektor ($a_0$) einer der folgenden Vektoren ist: ein willkürlich festgelegter Vektor, ein bei einer Initialisierung des medizinischen Geräts (1) bestimmter Vektor ($a_0$), ein zu bestimmten Zeitpunkten aus Daten des Beschleunigungssensors (2) bestimmter Vektor ($a_0$).

5. Medizinisches Gerät nach einem der vorhergehenden Ansprüche, **dadurch gekennzeichnet, dass** es sich bei dem mindestens einen Parameter um einen der folgenden Parameter handelt: eine Atemfrequenz, eine Länge einer Atemperiode, eine Schwankung einer Länge der Atemperiode, eine Länge einer Atempause.

6. Medizinisches Gerät nach einem der vorhergehenden Ansprüche, **dadurch gekennzeichnet, dass** das medizinische Gerät (1) ein implantierbares medizinisches Gerät ist (1).

7. Medizinisches Gerät nach einem der vorhergehenden Ansprüche, **dadurch gekennzeichnet, dass** das medizinische Gerät ein Gehäuse (4) aufweist, wobei der Beschleunigungssensor (2) und/oder die Auswertungseinheit (3) in einem durch das Gehäuse (4) gebildeten Innenraum (4a) angeordnet sind.

8. Verfahren zum Erfassen einer Atmung einer Person mittels eines medizinischen Gerätes (1) nach einem der vorhergehenden Ansprüche, aufweisend die Schritte:

    - Messen eines momentanen Beschleunigungsvektors ($a$(t)) mittels des Beschleunigungssensors (2), und
    - Bestimmen zumindest eines die Atmung der Person (P) charakterisierenden Parameters aus einer Änderung der Winkellage des momentanen Beschleunigungsvektors bezüglich eines Referenzvektors ($a_0$), wobei der Beschleunigungssensor (2) an oder in einem Oberkörper der Person (P) angeordnet ist.

9. Verfahren nach Anspruch 8, wobei zum Erfassen der Änderung der Winkellage eine der folgenden Größen mittels der Auswertungseinheit (3) wiederholt bestimmt wird: ein momentaner Winkel $\delta$(t) zwischen dem momentanen Beschleunigungsvektor ($a$(t)) und dem Referenzvektor ($a_0$), ein Kosinus des momentanen Winkels $\delta$(t) zwischen dem momentanen Beschleunigungsvektor ($a$(t)) und dem Referenzvektor ($a_0$), ein Quadrat des Kosinus des momentanen Winkels $\delta$(t) zwischen dem momentanen Beschleunigungsvektor ($a$(t)) und dem Referenzvektor ($a_0$).

10. Verfahren nach Anspruch 8 oder 9, wobei mittels der Auswertungseinheit (3) zum Bestimmen der jeweiligen Größe ein Skalarprodukt zwischen dem momentanen Beschleunigungsvektor ($a$(t)) und dem Referenzvektor ($a_0$) wiederholt berechnet wird.

FIG. 1

**FIG. 2**

cos(δ)

Zeit [s]

FIG. 3

EP 3 888 549 A1

EP 3 888 549 A1

Europäisches Patentamt
European Patent Office
Office européen des brevets

# EUROPÄISCHER RECHERCHENBERICHT

Nummer der Anmeldung

EP 20 16 7693

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2015 222499 B3 (TELOCATE GMBH [DE]) 19. Januar 2017 (2017-01-19) * Absätze [0001], [0020], [0021], [0042] - [0051], [0101]; Abbildung 7 * ----- | 1-10 | INV. A61B5/113 |
| X | US 2012/065524 A1 (MORREN GEERT GUY GEORGES [BE] ET AL) 15. März 2012 (2012-03-15) * Absätze [0003] - [0012], [0069] - [0096] * ----- | 1-10 | |
| X | JP 2007 292514 A (MATSUSHITA ELECTRIC WORKS LTD) 8. November 2007 (2007-11-08) * Absatz [0001] * ----- | 1,3-8,10 | |
| A | US 2013/116602 A1 (VAN DEN HEUVEL TEUN [NL] ET AL) 9. Mai 2013 (2013-05-09) * Absatz [0046] * ----- | 1-10 | |

|  | RECHERCHIERTE SACHGEBIETE (IPC) |
|---|---|
|  | A61N A61B |

Der vorliegende Recherchenbericht wurde für alle Patentansprüche erstellt

| Recherchenort | Abschlußdatum der Recherche | Prüfer |
|---|---|---|
| Berlin | 10. September 2020 | Trachterna, Morten |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 16 7693

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-09-2020

| Im Recherchenbericht angeführtes Patentdokument | Datum der Veröffentlichung | Mitglied(er) der Patentfamilie | | Datum der Veröffentlichung |
|---|---|---|---|---|
| DE 102015222499 B3 | 19-01-2017 | KEINE | | |
| US 2012065524 A1 | 15-03-2012 | CN | 102458246 A | 16-05-2012 |
| | | EP | 2263532 A1 | 22-12-2010 |
| | | EP | 2437662 A1 | 11-04-2012 |
| | | JP | 5784590 B2 | 24-09-2015 |
| | | JP | 2012528657 A | 15-11-2012 |
| | | RU | 2011152964 A | 20-07-2013 |
| | | US | 2012065524 A1 | 15-03-2012 |
| | | WO | 2010140130 A1 | 09-12-2010 |
| JP 2007292514 A | 08-11-2007 | JP | 4862469 B2 | 25-01-2012 |
| | | JP | 2007292514 A | 08-11-2007 |
| US 2013116602 A1 | 09-05-2013 | CN | 103025240 A | 03-04-2013 |
| | | EP | 2598028 A2 | 05-06-2013 |
| | | JP | 5847178 B2 | 20-01-2016 |
| | | JP | 2013532539 A | 19-08-2013 |
| | | US | 2013116602 A1 | 09-05-2013 |
| | | WO | 2012014110 A2 | 02-02-2012 |

EPO FORM P0461

Für nähere Einzelheiten zu diesem Anhang : siehe Amtsblatt des Europäischen Patentamts, Nr.12/82

**EP 3 888 549 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20090062628 A1
- US 20150342518 A1